# EUROPEAN PATENT APPLICATION

(11) **EP 4 851 186 A1**
(43) Date of publication of application: **09.12.2026**
(21) Application number: 25849692.6
(22) Date of filing: 17.06.2025
(51) Int. Cl.: C07K 14/725, C07K 16/28, C12N 15/12, A61K 40/11, A61P 35/00

(54) **T CELL RECEPTOR AND USE THEREOF**

(30) Priority: 10.04.2025 CN 202510445476
(71) Applicant: HRYZ (Guangzhou) Biotech Co., Ltd, Guangzhou, Guangdong 510700 (CN); HRYZ (Shanghai) Biotech Co., Ltd, Shanghai 201600 (CN)
(72) Inventor: HAN, Yanyan, Guangzhou, Guangdong 510700 (CN); CHU, Junjun, Guangzhou, Guangdong 510700 (CN); LEONG, Tein Foong, Guangzhou, Guangdong 510700 (CN); CHEN, Yaolong, Guangzhou, Guangdong 510700 (CN); DU, Jiajia, Guangzhou, Guangdong 510700 (CN); ZHANG, Lulu, Guangzhou, Guangdong 510700 (CN)
(74) Representative: ABG Intellectual Property Law, S.L.
(86) International application number: PCT/CN2025/101336
(87) International publication number: WO 2026/213956

(57) **Abstract**

The present invention relates to the field of immunotherapy for cancer. In particular, the present application relates in some aspects to binding molecules, such as complexes capable of specifically binding to major histocompatibility complex (MHC) molecules and AFP peptides. The present invention relates to T cell receptors (TCRs) that bind or recognize such peptide epitopes, including antigen binding domains thereof. The present application further relates to engineered cells comprising such binding molecules, e.g., TCR bispecific proteins or sTCRs, etc., and use thereof in immunotherapy for cancer.

## Description

### Technical Field

The present invention relates to the field of immunotherapy for cancer, and in particular, to a T cell receptor capable of specifically binding to a complex of major histocompatibility complex (MHC) molecules and AFP peptides and use thereof.

### Background Art

Immunotherapy allows for an effective cure of cancer by enhancing the function of a patient's immune system and using immune cells to remove cancer cells in the body. Cell therapy is an adoptive therapy in which T cells that are specifically reactive to a target cell antigen are transferred into a patient to act on the target cell. T cell receptors (TCRs) are membrane proteins on the surface of T cells that can recognize corresponding antigenic short peptides on the surface of target cells. In the immune system, T cells come into direct physical contact with antigen-presenting cells (APCs) upon the binding of TCRs that are specific for the antigenic short peptides to the short peptides- major histocompatibility complex (a pMHC complex), and then other cell membrane surface molecules of both the T cells and the APCs interact with each other to cause a series of subsequent signal transduction, so that T cells having different antigen specificities exert immune effects on their target cells.

Engineered T cells containing tumor antigen-specific T cell receptors (TCRs) can overcome some of the challenges existing in current adoptive lymphocyte therapies, since they can rapidly produce tumor-reactive T lymphocytes with defined antigen specificities. There is a need in that art for TCRs that target different tumor antigens and can provide high antitumor efficacy and low off-target effects *in vivo*. Common problems with TCR-T cell therapies include serious adverse events in clinical trials (such as toxicity in central nervous system), which may be associated with inappropriate selection of target (the so-called on-target off tumor effect) and biased expansion of a population of T cells.

Alpha-fetoprotein (AFP) is a highly specific protein expressed in liver cancer cells. Many patients with liver cancer (70-80%) are characterized by high expression of AFP gene during the morbidity of liver cancer. AFP is an embryogenic protein whose gene is open and expressed during fetal development, but it is in a substantially closed condition after two years of life. However, when liver cancer or benign liver regeneration occurs in adults, the gene of AFP is reactivated and abundantly expressed, which is considered as a clinically canonical tumor marker for liver cancer, and thus being taken as the gold standard for the diagnosis of liver cancer.

Human AFP belongs to α-globulin, running between albumin and globulin in electrophoresis, having a molecular weight of about 64,000-72,000 and precipitation coefficient of 4.5. This protein consists of about 18 amino acids, with carbohydrates comprising about 4% of the protein. AFP derived from a fetus exhibits consistent electrophoretic motion, and those from a tumor source comprise four variants or subcomponents, with slight difference in electrophoretic motion, which is mainly due to the content of carbohydrates rather than changes in peptide chains.

AFP-specific TCR and TCR-T are therefore promising to become cancer therapies with high antitumor efficacy and low off-target effects.

### Summary of the Invention

In order to achieve the above objective, the present invention discloses in a first aspect a T cell receptor that recognizes an HLA-A-restricted AFP₁₅₈₋₁₆₆ epitope peptide, comprising an alpha chain and a beta chain,
wherein the alpha chain and the beta chain comprise the following complementarity determining regions (CDRs):
1) CDR1, on the alpha chain, whose amino acid sequence is set forth in one of SEQ ID NOs: 7-12, CDR2, on the alpha chain, whose amino acid sequence is set forth in SEQ ID NO: 2, and CDR3, on the alpha chain, whose amino acid sequence is set forth in SEQ ID NO: 3;
   CDR1, on the beta chain, whose amino acid sequence is set forth in SEQ ID NO: 4, CDR2, on the beta chain, whose amino acid sequence is set forth in SEQ ID NO: 5, and CDR3, on the beta chain, whose amino acid sequence is set forth in SEQ ID NO: 6; or
2) CDR1, on the alpha chain, whose amino acid sequence is set forth in SEQ ID NO: 1, CDR2, on the alpha chain, whose amino acid sequence is set forth in SEQ ID NO: 2, and CDR3, on the alpha chain, whose amino acid sequence is set forth in SEQ ID NO: 3;
   CDR1, on the beta chain, whose amino acid sequence is set forth in SEQ ID NO: 4, CDR2, on the beta chain, whose amino acid sequence is set forth in SEQ ID NO: 14, and CDR3, on the beta chain, whose amino acid sequence is set forth in SEQ ID NO: 6; or
3) CDR1, on the alpha chain, whose amino acid sequence is set forth in SEQ ID NO: 1 or 7, CDR2, on the alpha chain, whose amino acid sequence is set forth in SEQ ID NO: 2, and CDR3, on the alpha chain, whose amino acid sequence is set forth in SEQ ID NO: 13;
   CDR1, on the beta chain, whose amino acid sequence is set forth in one of SEQ ID NOs: 4 and 15-17, CDR2, on the beta chain, whose amino acid sequence is set forth in SEQ ID NO: 5, 14 or 18, and CDR3, on the beta chain, whose amino acid sequence is set forth in SEQ ID NO: 6.

The present invention also protects a polypeptide or protein molecule having a similar function to that of the T cell receptor of the present invention, which is obtained by substitution and/or deletion and/or addition of one or more amino acid residues; or a protein having more than 90% sequence similarity and associated with the function of recognizing an HLA-A-restricted AFP₁₅₈₋₁₆₆ epitope peptide.

In the T cell receptor, it recognizes an HLA-A-restricted AFP₁₅₈₋₁₆₆ epitope peptide, and the amino acid sequence of the epitope peptide is set forth in SEQ ID NO: 19, as specifically shown below:
FMNKFIYEI (SEQ ID NO: 19).

Preferably, the typing of the HLA comprises HLA-A*0203, HLA-A*0201, HLA-A*0206, or HLA-A*0207.

The present invention discloses in a second aspect a nucleic acid molecule encoding the T cell receptor as described above.

In the present invention, it is well known to those skilled in the art that due to the degeneracy of genetic codes of a protein, there is in most cases more than one codon that dictates an amino acid, and substitution of the third nucleotide in a triplet codon tends not to change the composition of an amino acid, thus the nucleotide sequences of genes encoding the same protein may be different. Those skilled in the art can completely deduce the nucleotide sequences of the genes encoding an amino acid sequence (s) from the amino acid sequence(s) disclosed in the present invention according to a well-known codon table, and obtain the nucleotide sequences by a biological approach (such as a PCR process, or a mutation process) or a chemical synthesis approach, and those nucleotide sequences derived therefrom thus should be encompassed within the present invention.

The present invention discloses in a third aspect an engineered immune cell expressing the T cell receptor as described above.

Wherein, the engineered immune cell is obtained by introducing the T cell receptor into an immune cell by means of transduction with a viral vector or a non-viral vector, a CRISPR/Cas9 system, a transposon system, or transfection with mRNA.

The transduction with a viral vector can be done by transferring a TCR gene into a T cell via a retrovirus or lentivirus.

The transduction with a non-viral vector involves electroporation or nanoparticle technology by which a TCR gene is transferred into a T cell. The engineered immune cell is derived from a NK cell or a T cell.

The present invention discloses in a fourth aspect a TCR bispecific protein consisting of an antibody targeting a surface receptor molecule on a cell membrane of an immune cell, and an extracellular membrane region of the T cell receptor as described above, and binding to both an immune cell and a tumor cell; the immune cell comprises a T cell or a NK cell; the antibody against a surface receptor molecule on a cell membrane of an immune cell comprises an scFv or Fab antibody fragment.

The surface receptor molecule on a cell membrane of an immune cell is preferably CD3, CD4, CD8, CD16a, and/or NKG2D.

The extracellular membrane region of the T cell receptor recognizes and binds to an antigen on a tumor cell.

The present invention discloses in a fifth aspect an mRNA preparation of a TCR bispecific protein, which comprises the TCR bispecific protein as described above, and in particular, expresses a TCR bispecific protein *in vivo* using mRNA technology.

The present invention discloses in a sixth aspect use of the T cell receptor, the nucleic acid molecule, the engineered immune cell, the TCR bispecific protein, or the mRNA preparation of a TCR bispecific protein as described above in the manufacture of a medicament for preventing and/or treating a disease or condition expressing AFP.

In the use, the disease or condition expressing AFP comprises liver cancer, germ cell tumors, hepatitis, gastric cancer, and the like.
the medicament further comprises a TCR protein drug;
the TCR protein drug is prepared from a multispecific T cell complex prepared from the T cell receptor as described above;
wherein the multispecific T cell complex comprises a TCR bispecific antibody, a TCR trispecific antibody, and a TCR tetraspecific antibody;
and comprises a multispecific T cell complex formed from a T cell receptor, a TCR bispecific antibody, a TCR trispecific antibody, or a TCR tetraspecific antibody linked to a nuclear drug, a toxin, a fluorescein, and an antibody fragment.

### Brief Description of the Drawings

The following examples are intended to be purely exemplary of the present application, and should not be construed as limiting the present invention in any way. The follow examples and detail description are provided by way of illustration and not of limitation.
Fig. 1 shows an HLA restriction assay in a K562 cell line and an *in vitro* toxicity assay in HepG2 of T cells expressing AFP antigen-specific T cell receptors of AM14-M1.
Fig. 2 shows an HLA restriction assay in a K562 cell line and an *in vitro* toxicity assay in HepG2 of T cells expressing AFP antigen-specific T cell receptors of AM14-M2.
Fig. 3 shows an HLA restriction assay in a K562 cell line and an *in vitro* toxicity assay in HepG2 of T cells expressing AFP antigen-specific T cell receptors of AM14-M3.
Fig. 4 shows an HLA restriction assay in a K562 cell line and an *in vitro* toxicity assay in HepG2 of T cells expressing AFP antigen-specific T cell receptors of AM14-M4.
Fig. 5 shows an HLA restriction assay in a K562 cell line and an *in vitro* toxicity assay in HepG2 of T cells expressing AFP antigen-specific T cell receptors of AM14-M5.
Fig. 6 shows an HLA restriction assay of T cells having AFP antigen-specific T cell receptors expressing AM14-M6 in a K562 cell line.
Fig. 7 shows an HLA restriction assay of T cells having AFP antigen-specific T cell receptors expressing AM14-M7 in a K562 cell line.
Fig. 8 shows an HLA restriction assay in a K562 cell line and an *in vitro* toxicity assay in HepG2 of T cells expressing AFP antigen-specific T cell receptors of AM14-M8.
Fig. 9 shows an AFP-TCR bispecific protein, AM14-M7, efficiently inducing T cells to recognize the AFP-HLA-A*02:03 complex on the membranes of target cells and activating T cells to secrete IFN-γ.
Fig. 10 shows AFP-TCR bispecific proteins, AM14-M10-13, efficiently inducing T cells to recognize the AFP-HLA-A*02:03 complex on the membranes of target cells and activating T cells to express luciferase, Luciferase.
Fig. 11 shows AFP-TCR bispecific proteins, AM14-M14-18, efficiently inducing T cells to recognize the AFP-HLA-A*02:03 complex on the membranes of target cells and activating T cells to express luciferase, Luciferase.

### Detailed Description of Examples

The following examples are intended to be purely exemplary of the present application, and should not be construed as limiting the present invention in any way. The follow examples and detail description are provided by way of illustration and not of limitation.

### Example 1: Preparation of AFP-specific T cells

TCR-T was prepared by preparation of TCR mRNA and electrotransformation of the activated CD3-T cells. On Day 1, PBMC was subjected to recovery, and pan-T sorting magnetic beads were added for incubation at 4°C for 5 min, followed by addition of a secondary antibody for incubation at 4°C for 10 min. The cell suspension was added to a sorting column, and CD3-T cells were sorted in a magnetic field. The sorted CD3-T cells were activated by T cell Transact (10 µL/1E6 cells), and the cell density was adjusted to 1×10⁶ cells/mL using an AIM-V medium. On Day 7, the activated CD3-T cells were harvested and centrifuged at 350 g for 5 minutes, the supernatant was removed and a fresh medium was added, and the cells were sampled for counting. The cells were washed with PBS, and a solution for electrotransformation (1E6 cells/20 µL of the solution for electrotransformation) was added for resuspension after removal of the supernatant. 20 µL of the cell suspension was taken and added to EP tubes for a total of 8 samples, to which 2 µL of each TCR mRNA (AM14-M1, AM14-M2, AM14-M3, AM14-M4, AM14-M5, AM14-M6, AM14-M7, and AM14-M8 mRNA) was added and mixed up, followed by loading to the small tanks of a vessel for electrotransformation in a 20 µL system. The vessel for electrotransformation was gently tapped to ensure that the samples had covered the bottom of the vessel, which was then placed in the tank of the electroporator for electrotransformation under a set program.
Wild-type AM14 comprises:
   alpha chain, CDR1: VSGLRG (SEQ ID NO: 1)
   CDR2: LYSAGEE (SEQ ID NO: 2)
   CDR3: CAVQADSNSGYALNF (SEQ ID NO: 3)
   beta chain, CDR1: SEHNR (SEQ ID NO: 4)
   CDR2: FQNEAQ (SEQ ID NO: 5)
   CDR3: CASSLTGLEQFF (SEQ ID NO: 6).
AM14-M1 comprises:
   alpha chain, CDR1: SR MLRG (SEQ ID NO: 7)
   CDR2: LYSAGEE (SEQ ID NO: 2)
   CDR3: CAVQADSNSGYALNF (SEQ ID NO: 3)
   beta chain, CDR1: SEHNR (SEQ ID NO: 4)
   CDR2: FQNEAQ (SEQ ID NO: 5)
   CDR3: CASSLTGLEQFF (SEQ ID NO: 6).
AM14-M2 comprises:
   alpha chain, CDR1: SSMLRG (SEQ ID NO: 8)
   CDR2: LYSAGEE (SEQ ID NO: 2)
   CDR3: CAVQADSNSGYALNF (SEQ ID NO: 3)
   beta chain, CDR1: SEHNR (SEQ ID NO: 4)
   CDR2: FQNEAQ (SEQ ID NO: 5)
   CDR3: CASSLTGLEQFF (SEQ ID NO: 6).
AM14-M3 comprises:
   alpha chain, CDR1: STLLRG (SEQ ID NO: 9)
   CDR2: LYSAGEE (SEQ ID NO: 2)
   CDR3: CAVQADSNSGYALNF (SEQ ID NO: 3)
   beta chain, CDR1: SEHNR (SEQ ID NO: 4)
   CDR2: FQNEAQ (SEQ ID NO: 5)
   CDR3: CASSLTGLEQFF (SEQ ID NO: 6).
AM14-M4 comprises:
   alpha chain, CDR1: SSLLRG (SEQ ID NO: 10)
   CDR2: LYSAGEE (SEQ ID NO: 2)
   CDR3: CAVQADSNSGYALNF (SEQ ID NO: 3)
   beta chain, CDR1: SEHNR (SEQ ID NO: 4)
   CDR2: FQNEAQ (SEQ ID NO: 5)
   CDR3: CASSLTGLEQFF (SEQ ID NO: 6).
AM14-M5 comprises:
   alpha chain, CDR1: HSLLRG (SEQ ID NO: 11)
   CDR2: LYSAGEE (SEQ ID NO: 2)
   CDR3: CAVQADSNSGYALNF (SEQ ID NO: 3)
   beta chain, CDR1: SEHNR (SEQ ID NO: 4)
   CDR2: FQNEAQ (SEQ ID NO: 5)
   CDR3: CASSLTGLEQFF (SEQ ID NO: 6).
AM14-M6 comprises:
   alpha chain, CDR1: VSLLRG (SEQ ID NO: 12)
   CDR2: LYSAGEE (SEQ ID NO: 2)
   CDR3: CAVQADSNSGYALNF (SEQ ID NO: 3)
   beta chain, CDR1: SEHNR (SEQ ID NO: 4)
   CDR2: FQNEAQ (SEQ ID NO: 5)
   CDR3: CASSLTGLEQFF (SEQ ID NO: 6).
AM14-M7 comprises:
   alpha chain, CDR1: VSGLRG (SEQ ID NO: 1)
   CDR2: LYSAGEE (SEQ ID NO: 2)
   CDR3: CAVQADSWPSYALNF (SEQ ID NO: 13)
   beta chain, CDR1: SEHNR (SEQ ID NO: 4)
   CDR2: FQNEAQ (SEQ ID NO: 5)
   CDR3: CASSLTGLEQFF (SEQ ID NO: 6).
AM14-M8 comprises:
   alpha chain, CDR1: VSGLRG (SEQ ID NO: 1)
   CDR2: LYSAGEE (SEQ ID NO: 2)
   CDR3: CAVQADSNSGYALNF (SEQ ID NO: 3)
   beta chain, CDR1: SEHNR (SEQ ID NO: 4)
   CDR2: FIFGAQ (SEQ ID NO: 14)
   CDR3: CASSLTGLEQFF (SEQ ID NO: 6).

### Example 2: Verification of AFP-specific T cell receptor, AM14-M1

Determination of TCR Expression and Polypeptide Recognition

K562 cells having a genotype of HLA-A*02:03/HLA-A*02:01/HLA-A*02:06/HLA-A*02:07 each were, respectively, loaded with an AFP₁₅₈₋₁₆₆ polypeptide to stimulate T cells transfected with T cell receptors in an HLA restriction assay, and measured by Flow Cytometry and Human IFN-γ ELISA.

TCR-transduced T cells were harvested, washed by the addition of 2 mL of PBS, and centrifuged at 350 g for 5 minutes, and the supernatant was sucked out completely. TCR-transduced T cells were then resuspended and adjusted to 1×10⁶ cells/mL by the addition of an AIM-V medium containing 5% AB serum, and 100 µL was added to 96-well plates. Target cells loaded with an AFP₁₅₈₋₁₆₆ polypeptide, at the same density and having the same number of cells, were mixed with tumor-specific T cells. Brefeldin A (at a final concentration of 3 µg/mL) was added to the wells and cultured for 4 hours. After 4 hours, cells were harvested and used for intracellular IFN-γ, subjected to staining for surface CD3 and TCR expression, and detected by Flow Cytometry.

For the detection of IFN-γ by ELISA, 100 µL of K562 cells which are loaded with an AFP₁₅₈₋₁₆₆ polypeptide per well (1×10⁶ cells/mL), were mixed with 100 µL of TCR-transduced T cells per well (1×10⁶ cells/mL) in the 96-well plates, and cultured in an incubator for 24 hours. After 24 hours, 175 µL of supernatant was harvested in each well in the 96-well plates and used for the detection of IFN-γ by ELISA, using IFN-γ ELISA HRP Kit. On Day 1, ELISA plates with high protein binding were coated (by addition of 100 µL/well) with Antibody 1-D1K (IFN-γ ELISA HRP Kit) which have been diluted to 2 µg/mL in PBS (pH 7.4), and incubated overnight at 4°C. On Day 2, the plates were washed twice with PBS (200 µL/well). The plates were blocked by the addition of 200 µL of a medium to each well, and cultured at room temperature (RT) for 1 hour. Human IFN-γ standard (IFN-γ ELISA HRP Kit) was prepared in 2 mL of PBS (with 1% BSA) to a concentration of 0.5 µg/mL and kept at RT for 15 minutes, followed by vortex shaking a test tube. 100 µL of sample or standard per well was diluted in a medium and cultured at RT for 2 hours. The sample and standard probe were tested in duplicate. The plates were washed five times with PBS containing 0.05% Tween 20. 100 µL of Antibody 7-B6-1-Biotin (IFN-γ ELISA HRP Kit) at 1 µg/mL (in PBS) was added to each well, cultured at RT for 1 hour, and washed. 100 µL of Streptavidin-HRP (IFN-γ ELISA HRP Kit) that has een diluted in PBS at 1:1000 was added to each well, cultured at RT for 1 hour, and washed. 100 µL of a TMB substrate solution was added, and cultured at RT in the dark for 15 to 30 minutes until the solution in the well visibly turned blue. 100 µL of a stop solution was added to each well to stop the enzymatic reaction. The color of the solution changed from blue to yellow. Light intensity was measured at 450 nm using an ELISA reader.

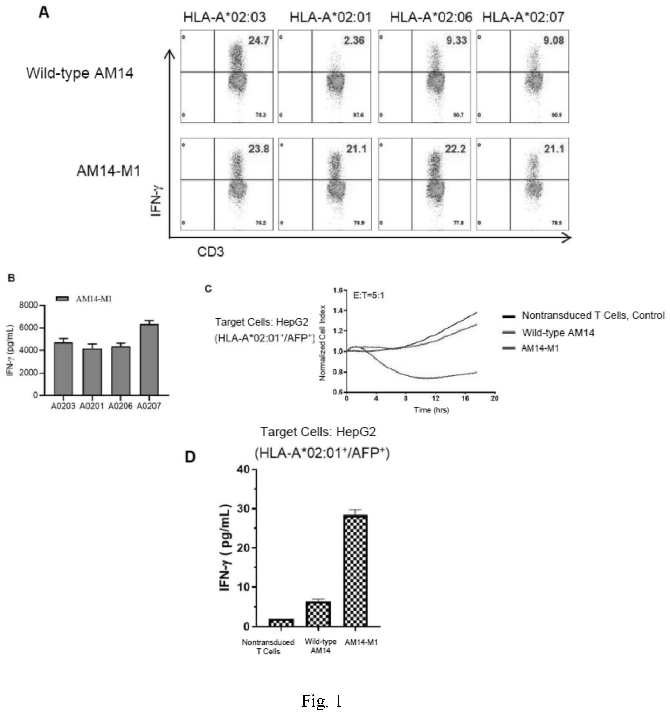

As shown in Panels A and B in Fig. 1, T cells transfected with AM14-M1 could recognize the AFP₁₅₈₋₁₆₆ antigenic peptide presented by HLA-A*02:03/HLA-A*02:01/HLA-A*02:06/HLA-A*02:07.

### In vitro Toxicity Assay

T cells transfected with a T cell receptor, AM14-M1, were used as effector cells, and mixed with target cells in a ratio of 5:1 as an experimental group. The target cells used in this experiment were HepG2 cells that naturally expressed HLA-A*02:01 and AFP protein. T cells that were not transfected with the T cell receptor were mixed with target cells in the same ratio as a control group. Killing of target cells by effector cells from the experimental group and the control group was continuously measured for 20 hours. After 20 hours, the plate for killing was removed and centrifuged at 500 g, and 175 µL of the supernatant was harvested per well and used for the detection of IFN-γ by ELISA, using IFN-γ ELISA HRP Kit.

As shown in Panels C and D in Fig. 1, T cells transfected with AM14-M1 had a significant killing effect on HepG2 target cells that naturally expressed AFP protein, with IFN-γ secreted, and the target cells were killed at a Effector to Target ratio (E:T) of 5:1. Nontransfected T cells in the control group had no killing effect on the target cells.

### Example 3: Verification of AFP-specific T cell Receptors, AM14-M2-8

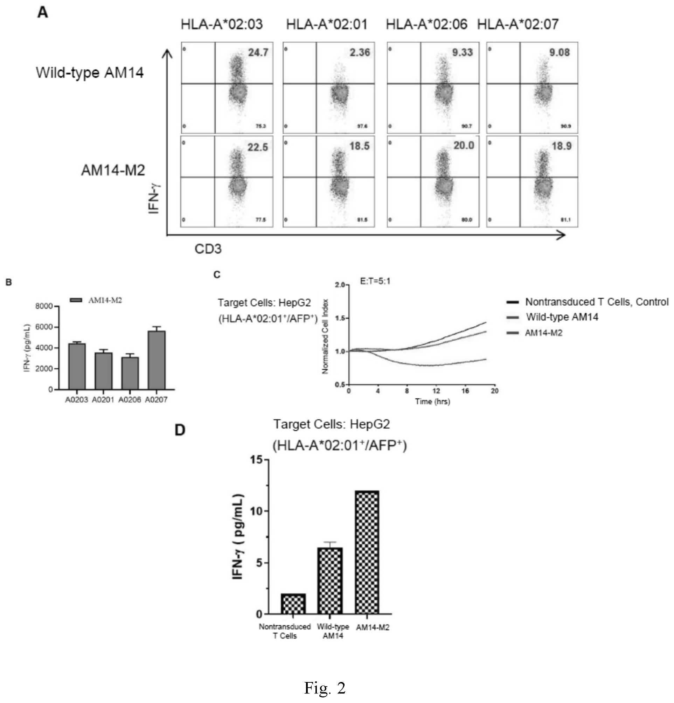

AFP-specific T cell receptors, AM14-M2-8, were assayed in the same progress as described in Example 2 for determination of TCR expression and polypeptide recognition, HLA restriction assay, *in vitro* toxicity assay, and detection of IFN-γ by ELISA. The results were shown in Figs. 2-8.

Panels A and B in Fig. 2 showed that T cells transfected with AM14-M2 could recognize the AFP₁₅₈₋₁₆₆ antigenic peptide presented by HLA-A*02:03/HLA-A*02:01/HLA-A*02:06/HLA-A*02:07; panels C and D in Fig. 2 showed that T cells transfected with AM14-M2 had a significant killing effect on HepG2 target cells that naturally expressed AFP protein, with the target cells killed; the Effector to Target ratio (E:T) was 5:1.

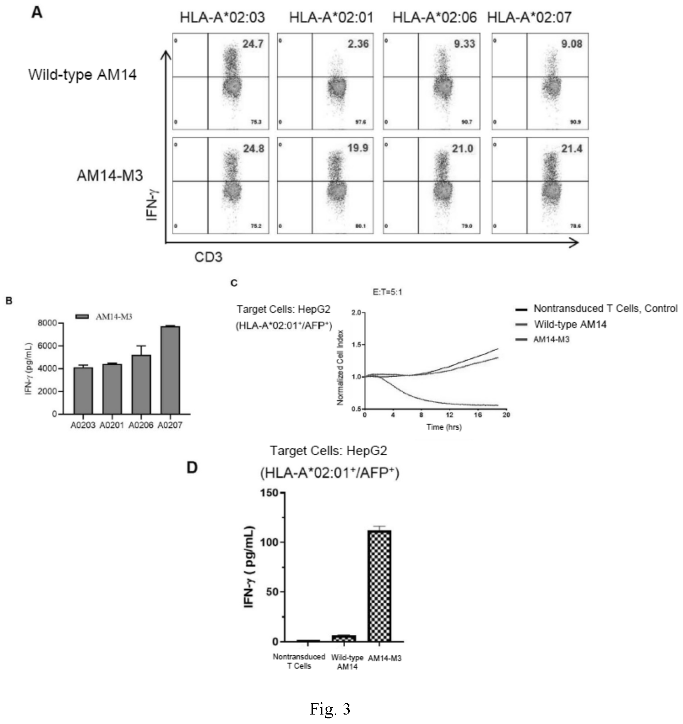

Panels A and B in Fig. 3 showed that T cells transfected with AM14-M3 could recognize the AFP₁₅₈₋₁₆₆ antigenic peptide presented by HLA-A*02:03/HLA-A*02:01/HLA-A*02:06/HLA-A*02:07; panels C and D in Fig. 3 showed that T cells transfected with AM14-M3 had a significant killing effect on HepG2 target cells that naturally expressed AFP protein, with the target cells killed; the Effector to Target ratio (E:T) was 5:1.

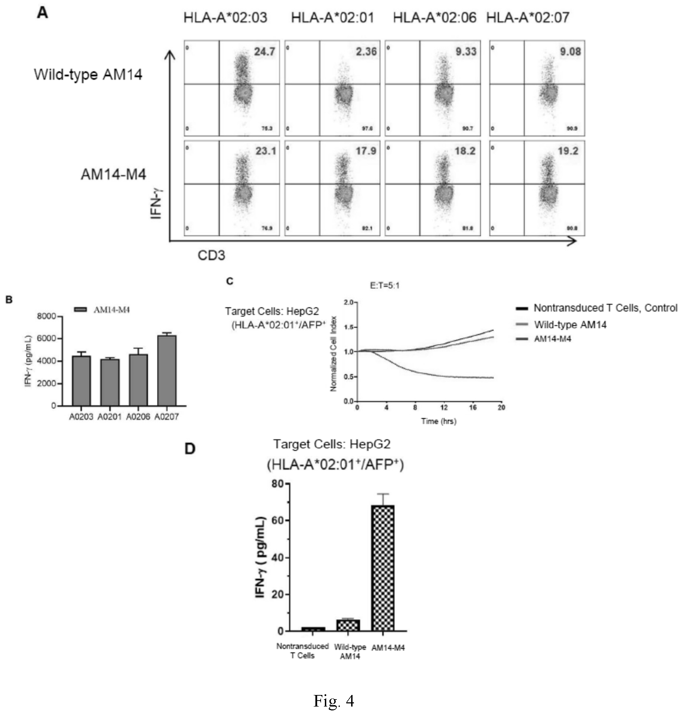

Panels A and B in Fig. 4 showed that T cells transfected with AM14-M4 could recognize the AFP₁₅₈₋₁₆₆ antigenic peptide presented by HLA-A*02:03/HLA-A*02:01/HLA-A*02:06/HLA-A*02:07; panels C and D in Fig. 4 showed that T cells transfected with AM14-M4 had a significant killing effect on HepG2 target cells that naturally expressed AFP protein, with the target cells killed; the Effector to Target ratio (E:T) was 5:1.

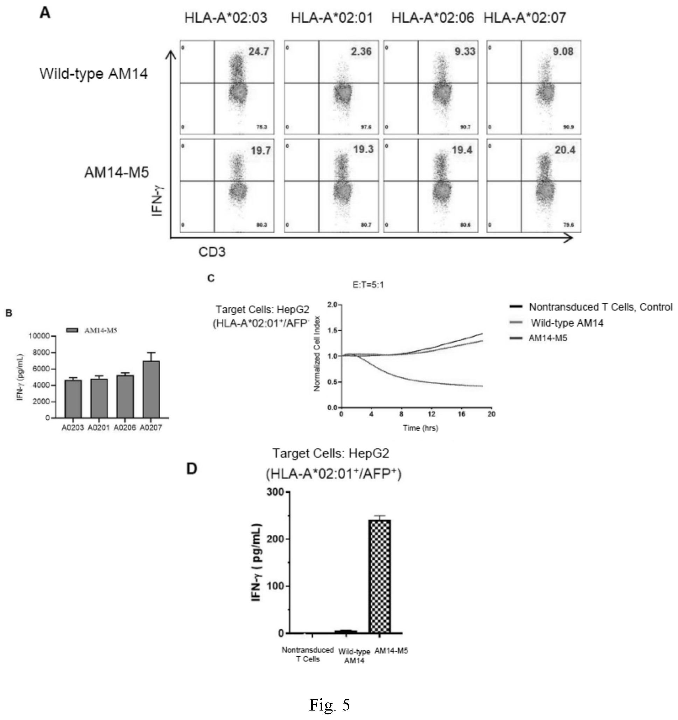

Panels A and B in Fig. 5 showed that T cells transfected with AM14-M5 could recognize the AFP₁₅₈₋₁₆₆ antigenic peptide presented by HLA-A*02:03/HLA-A*02:01/HLA-A*02:06/HLA-A*02:07; panels C and D in Fig. 5 showed that T cells transfected with AM14-M5 had a significant killing effect on HepG2 target cells that naturally expressed AFP protein, with the target cells killed; the Effector to Target ratio (E:T) was 5:1.

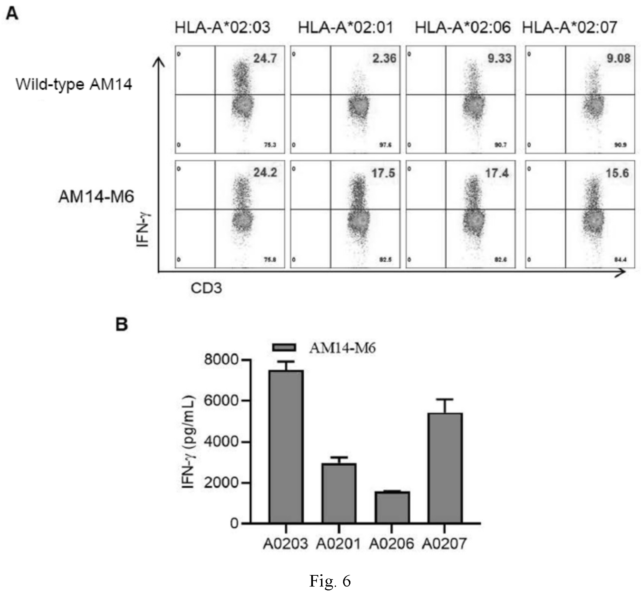

Panels A and B in Fig. 6 showed that T cells transfected with AM14-M6 could recognize the AFP₁₅₈₋₁₆₆ antigenic peptide presented by HLA-A*02:03/HLA-A*02:01/HLA-A*02:06/HLA-A*02:07.

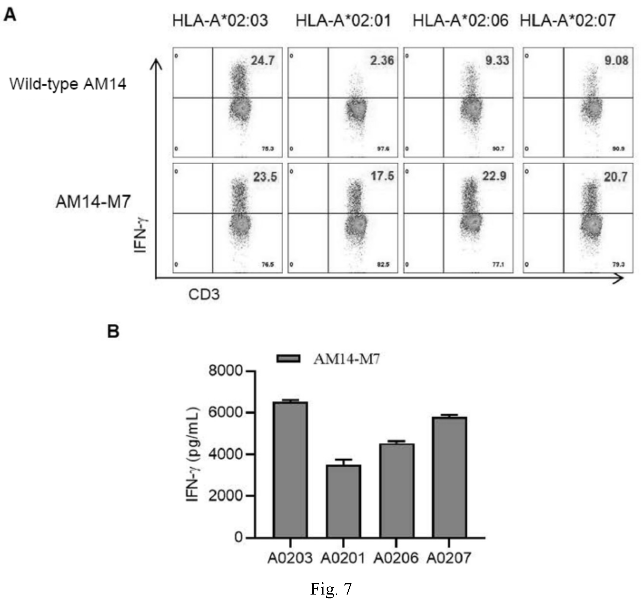

Panels A and B in Fig. 7 showed that T cells transfected with AM14-M7 could recognize the AFP₁₅₈₋₁₆₆ antigenic peptide presented by HLA-A*02:03/HLA-A*02:01/HLA-A*02:06/HLA-A*02:07.

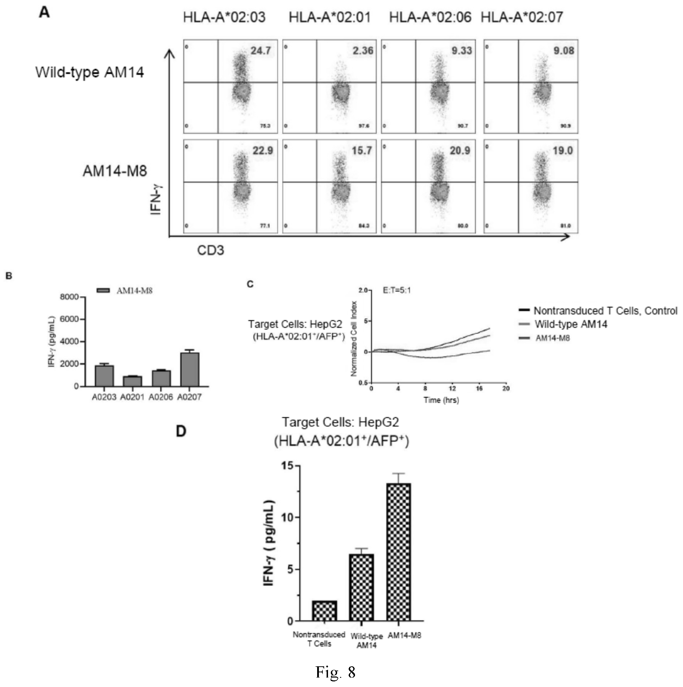

Panels A and B in Fig. 8 showed that T cells transfected with AM14-M8 could recognize the AFP₁₅₈₋₁₆₆ antigenic peptide presented by HLA-A*02:03/HLA-A*02:01/HLA-A*02:06/HLA-A*02:07; panels C and D in Fig. 8 showed that T cells transfected with AM14-M8 had a significant killing effect on HepG2 target cells that naturally expressed AFP protein, with the target cells killed; the Effector to Target ratio (E:T) was 5:1.

### Example 4: Verification of AFP-TCR Bispecific Protein

The objective of this example is to verify the recognition of target cells and epitopes by AFP-TCR bispecific protein, as well as functional detection of T cells induction *in vitro*.

Ten AFP antigen-specific T cell receptors, AM14-M7, AM14-M10, AM14-M11, AM14-M12, AM14-M13, AM14-M14, AM14-M15, AM14-M16, AM14-M17 and AM14-M18 were prepared for *in vitro* functional verification experiments.

### Detection of IFN-γ by ELISA

Activated T cells having HLA-A*02:03 as a genotype were co-cultured with AFP₁₅₈₋₁₆₆ antigenic peptide-loaded HLA-A*02:03 transduced K562 cells, at a ratio of 1:1, and a bispecific protein to be detected for 48 hours, followed by human IFN-γ measurement by ELISA.

On Day 1, HLA-A*02:03 transduced K562 cell were harvested, centrifuged at 350 g for 5 minutes, and the supernatant was discarded. The cell pellets were resuspended and adjusted to approximately 1×10⁶ cells/mL by the addition of a complete 1640 medium (1640 medium + 10% fetal bovine serum + 1% double antibiotics) and incubated withan AFP₁₅₈₋₁₆₆ antigenic peptide added at 1 µg/mL for 2 hours. AFP₁₅₈₋₁₆₆ antigenic peptide-loaded HLA-A*02:03 transduced K562 cells, and HLA-A*02:03 transduced K562 cells (negative control group) were harvested, centrifuged at 350 g for 5 minutes, and the supernatant was discarded. The pellets were washed with 10 mL of PBS (containing 2% fetal bovine serum) and centrifuged at 350 g for 5 minutes, and the supernatant was discarded. The cell pellets were resuspended and adjusted to 1×10⁶ cells/mL by the addition of a complete AIM-V medium (AIM-V medium + 2% human serum + 1% double antibiotics), and 100 µL was added to each well in 96-well plates with round bottoms. Activated T cells were harvested and centrifuged at 350 g for 5 minutes, and the supernatant was discarded. The cell pellets were resuspended and adjusted to 1×10⁶ cells/mL by the addition of a complete AIM-V medium (AIM-V medium + 2% human serum + 1% double antibiotics), and a bispecific protein at a protein concentration of 20 nM was added (at a final concentration of 10 nM upon dilution). After mixing them well, AFP₁₅₈₋₁₆₆ antigenic peptide-loaded HLA-A*02:03 transduced K562 cells, and HLA-A*02:03 transduced K562 cells were added at 100 µL/well, mixed well, and cultured for 48 hours. On Day 2, ELISA plates with high protein binding were coated (by addition of 100 µL/well) with Antibody 1-D1K (IFN-γ ELISA HRP Kit) that has been diluted to 2 µg/mL in PBS (pH 7.4), and incubated overnight at 4°C. On Day 3, the ELISA plates were washed twice with PBS (200 µL/well). The ELISA plates were blocked by the addition of 200 µL of culture medium to each well, and incubated at room temperature (RT) for 1 hour. Human IFN-γ standard (IFN-γ ELISA HRP Kit) was prepared in 2 mL of PBS (with 1% BSA) to a concentration of 0.5 µg/mL and kept at RT for 15 minutes, followed by vortex shaking in a test tube. 100 µL of sample or standard per well was diluted in culture medium and incubated at RT for 2 hours. The ELISA plates were washed five times with PBS containing 0.05% Tween 20. 100 µL of Antibody 7-B6-1-Biotin (IFN-γ ELISA HRP Kit) was added to each well at 1 µg/mL (in PBS), incubated at RT for 1 hour, and washed. 100 µL of Streptavidin-HRP (IFN-γ ELISA HRP Kit) that has been diluted in PBS at 1:1000 was added to each well, incubated at RT for 1 hour, and washed. 100 µL of TMB substrate solution was added to each cell, and incubated at RT in the dark for 15 to 30 minutes until the solution in the well visibly turned blue. 100 µL of stop solution was added to each well to stop the enzymatic reaction. The color of the solution changed from blue to yellow. Light intensity was measured at 450 nm using an ELISA reader. The final measurement value was a result obtained by subtracting a measurement value of the negative control group with a measurement value of the experimental group .

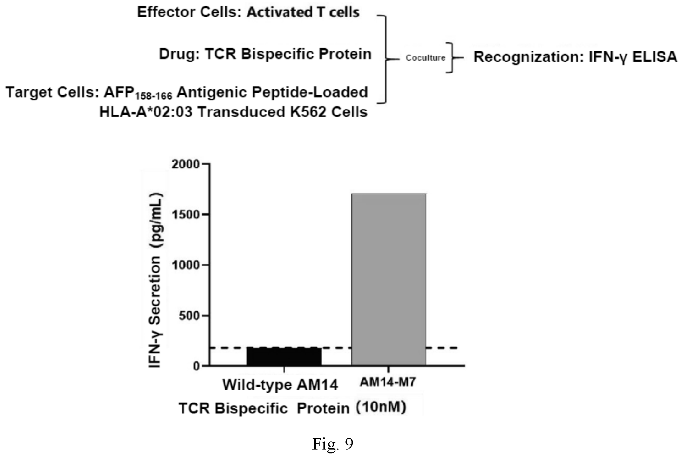

As shown in Fig. 9, AM14-M7 at 10 nM concentration of the TCR bispecific protein, functioned to induce a stronger and more significant T cell activation and IFN-γ secretion than wild-type AM14 .

### Jurkat Luciferase Assay

AFP₁₅₈₋₁₆₆ antigenic peptide-loaded HLA-A*02:03 transduced K562 cells, and HLA-A*02:03 transduced K562 cells were cocultured with Jurkat Luciferase cells, at a ratio of 1:1 , and a bispecific protein to be detected, respectively, for 24 hours, followed by the detection of Luciferase expression.

On Day 1, HLA-A*02:03 transduced K562 cell were harvested, centrifuged at 350 g for 5 minutes, and the supernatant was discarded. The cell pellets were resuspended and adjusted to approximately 1×10⁶ cells/mL by the addition of a complete 1640 medium (1640 medium + 10% fetal bovine serum + 1% double antibiotics), and incubated with an AFP₁₅₈₋₁₆₆ antigenic peptide added at 1 µg/mL for 2 hours. AFP₁₅₈₋₁₆₆ antigenic peptide-loaded HLA-A*02:03 transduced K562 cells, and HLA-A*02:03 transduced K562 cells (negative control group) were harvested, centrifuged at 350 g for 5 minutes, and the supernatant was discarded. The pellets were washed with 10 mL of PBS (containing 2% fetal bovine serum) and centrifuged at 350 g for 5 minutes, and the supernatant was discarded. The cell pellets were resuspended and adjusted to 4×10⁵ cells/mL by the addition of a complete 1640 medium (1640 medium + 10% fetal bovine serum + 1% double antibiotics), and 50 µL was added to each well in 96-well plates with round bottoms. Jurkat Luciferase cells were harvested and centrifuged at 350 g for 5 minutes, and the supernatant was discarded. The cell pellets were resuspended and adjusted to 4×10⁵ cells/mL by the addition of a complete 1640 medium (1640 medium + 10% fetal bovine serum + 1% double antibiotics), and a bispecific protein was added at a protein concentration of 2 nM (at a final concentration of 1 nM upon dilution). After mixing them well, AFP₁₅₈₋₁₆₆ antigenic peptide-loaded HLA-A*02:03 transduced K562 cells, and HLA-A*02:03 transduced K562 cells were added at 50 µL/well, mixed well, and cultured for 24 hours. On Day 2, the detection reagent in a Firefly Luciferase Reporter Gene Assay Kit was brought back to room temperature in advance. The liquid cell mixture following coculture was blown for a homogenized condition, and 60 µL of the cell suspension was taken to a well of a white-colored ELISA plate. Then, 60 µL of the detection reagent was mixedwith the cell suspension for a full reaction for 10-20mins, and the fluorescence value was measured through chemical luminescence on a microplate reader. The final measurement value was obtained by subtracting the measurement value of the negative control group with the measurement value of the experimental group.

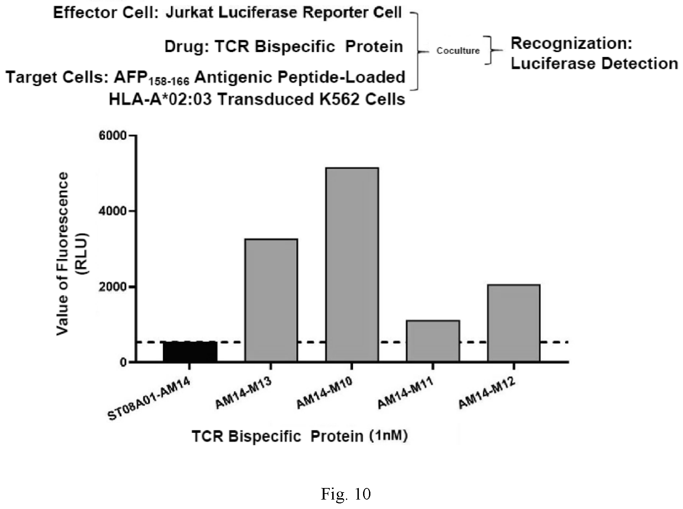

As shown in Fig. 10, AM14-M10, AM14-M11, AM14-M12 and AM14-M13, at 1 nM concentration of the TCR bispecific protein, functioned to induce a stronger and more significant T cell activation and Luciferase secretion than wild-type AM14.

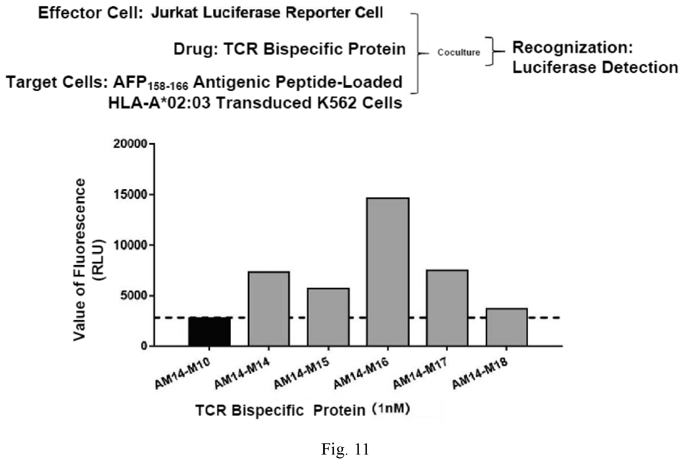

As shown in Fig. 11, AM14-M14, AM14-M15, AM14-M16, AM14-M17 and AM14-M18 at 1 nM concentration of the TCR bispecific antibody functioned to induce a stronger and more significant T cell activation and Luciferase secretion than AM14-M10.
AM14-M7 comprises:
   alpha chain, CDR1: VSGLRG (SEQ ID NO: 1)
   CDR2: LYSAGEE (SEQ ID NO: 2)
   CDR3: CAVQADSWPSYALNF (SEQ ID NO: 13)
   beta chain, CDR1: SEHNR (SEQ ID NO: 4)
   CDR2: FQNEAQ (SEQ ID NO: 5)
   CDR3: CASSLTGLEQFF (SEQ ID NO: 6).
AM14-M10 comprises:
   alpha chain, CDR1: SRMLRG (SEQ ID NO: 7)
   CDR2: LYSAGEE (SEQ ID NO: 2)
   CDR3: CAVQADSWPSYALNF (SEQ ID NO: 13)
   beta chain, CDR1: SEHNR (SEQ ID NO: 4)
   CDR2: FQNEAQ (SEQ ID NO: 14)
   CDR3: CASSLTGLEQFF (SEQ ID NO: 6).
AM14-M11 comprises:
   alpha chain, CDR1: VSGLRG (SEQ ID NO: 1)
   CDR2: LYSAGEE (SEQ ID NO: 2)
   CDR3: CAVQADSWPSYALNF (SEQ ID NO: 13)
   beta chain, CDR1: LGMNR (SEQ ID NO: 15)
   CDR2: FIFGAQ (SEQ ID NO: 14)
   CDR3: CASSLTGLEQFF (SEQ ID NO: 6).
AM14-M12 comprises:
   alpha chain, CDR1: VSGLRG (SEQ ID NO: 1)
   CDR2: LYSAGEE (SEQ ID NO: 2)
   CDR3: CAVQADSWPSYALNF (SEQ ID NO: 13)
   beta chain, CDR1: YDHNR (SEQ ID NO: 16)
   CDR2: FQNDYL (SEQ ID NO: 18)
   CDR3: CASSLTGLEQFF (SEQ ID NO: 6).
AM14-M13 comprises:
   alpha chain, CDR1: SRMLRG (SEQ ID NO: 7)
   CDR2: LYSAGEE (SEQ ID NO: 2)
   CDR3: CAVQADSWPSYALNF (SEQ ID NO: 13)
   beta chain, CDR1: LGMNR (SEQ ID NO: 15)
   CDR2: FIFGAQ (SEQ ID NO: 14)
   CDR3: CASSLTGLEQFF (SEQ ID NO: 6).
AM14-M14 comprises:
   alpha chain, CDR1: SRMLRG (SEQ ID NO: 7)
   CDR2: LYSAGEE (SEQ ID NO: 2)
   CDR3: CAVQADSWPSYALNF (SEQ ID NO: 13)
   beta chain, CDR1: LGMNR (SEQ ID NO: 15)
   CDR2: FQNEAQ (SEQ ID NO: 5)
   CDR3: CASSLTGLEQFF (SEQ ID NO: 6).
AM14-M15 comprises:
   alpha chain, CDR1: SRMLRG (SEQ ID NO: 7)
   CDR2: LYSAGEE (SEQ ID NO: 2)
   CDR3: CAVQADSWPSYALNF (SEQ ID NO: 13)
   beta chain, CDR1: YDHNR (SEQ ID NO: 16)
   CDR2: FQNEAQ (SEQ ID NO: 5)
   CDR3: CASSLTGLEQFF (SEQ ID NO: 6).
AM14-M16 comprises:
   alpha chain, CDR1: SRMLRG (SEQ ID NO: 7)
   CDR2: LYSAGEE (SEQ ID NO: 2)
   CDR3: CAVQADSWPSYALNF (SEQ ID NO: 13)
   beta chain, CDR1: SEYRW (SEQ ID NO: 17)
   CDR2: FQNEAQ (SEQ ID NO: 5)
   CDR3: CASSLTGLEQFF (SEQ ID NO: 6).
AM14-M17 comprises:
   alpha chain, CDR1: SRMLRG (SEQ ID NO: 7)
   CDR2: LYSAGEE (SEQ ID NO: 2)
   CDR3: CAVQADSWPSYALNF (SEQ ID NO: 13)
   beta chain, CDR1: SEHNR (SEQ ID NO: 4)
   CDR2: FIFGAQ (SEQ ID NO: 14)
   CDR3: CASSLTGLEQFF (SEQ ID NO: 6).
AM14-M18 comprises:
   alpha chain, CDR1: SRMLRG (SEQ ID NO: 7)
   CDR2: LYSAGEE (SEQ ID NO: 2)
   CDR3: CAVQADSWPSYALNF (SEQ ID NO: 13)
   beta chain, CDR1: SEHNR (SEQ ID NO: 4)
   CDR2: FQNDYL (SEQ ID NO: 18)
   CDR3: CASSLTGLEQFF (SEQ ID NO: 6).

## Claims

1. A T cell receptor comprising an alpha chain and a beta chain, **characterized in that**
wherein the alpha chain and the beta chain comprise the following complementarity determining regions (CDRs):
1) CDR1, on the alpha chain, whose amino acid sequence is set forth in one of SEQ ID NOs: 7-12, CDR2, on the alpha chain, whose amino acid sequence is set forth in SEQ ID NO: 2, and CDR3, on the alpha chain, whose amino acid sequence is set forth in SEQ ID NO: 3;
CDR1, on the beta chain, whose amino acid sequence is set forth in SEQ ID NO: 4, CDR2, on the beta chain, whose amino acid sequence is set forth in SEQ ID NO: 5, and CDR3, on the beta chain, whose amino acid sequence is set forth in SEQ ID NO: 6; or
2) CDR1, on the alpha chain, whose amino acid sequence is set forth in SEQ ID NO: 1, CDR2, on the alpha chain, whose amino acid sequence is set forth in SEQ ID NO: 2, and CDR3, on the alpha chain, whose amino acid sequence is set forth in SEQ ID NO: 3;
CDR1, on the beta chain, whose amino acid sequence is set forth in SEQ ID NO: 4, CDR2, on the beta chain, whose amino acid sequence is set forth in SEQ ID NO: 14, and CDR3, on the beta chain, whose amino acid sequence is set forth in SEQ ID NO: 6; or
3) CDR1, on the alpha chain, whose amino acid sequence is set forth in SEQ ID NO: 1 or 7, CDR2, on the alpha chain, whose amino acid sequence is set forth in SEQ ID NO: 2, and CDR3, on the alpha chain, whose amino acid sequence is set forth in SEQ ID NO: 13;
CDR1, on the beta chain, whose amino acid sequence is set forth in one of SEQ ID NOs: 4 and 15-17, CDR2, on the beta chain, whose amino acid sequence is set forth in SEQ ID NO: 5, 14 or 18, and CDR3, on the beta chain, whose amino acid sequence is set forth in SEQ ID NO: 6.

2. The T cell receptor according to claim 1, **characterized in that**, the T cell receptor recognizes an HLA-A-restricted AFP₁₅₈₋₁₆₆ epitope peptide whose amino acid sequence is set forth in SEQ ID NO: 19.

3. The T cell receptor according to claim 2, **characterized in that**, the HLA is typed as HLA-A, the HLA-A is HLA-A*0203, HLA-A*0201, HLA-A*0206, or HLA-A*0207.

4. A nucleic acid molecule encoding the T cell receptor according to any of claims 1-3.

5. An engineered immune cell, **characterized in that**, it expresses the T cell receptor of any of claims 1-3.

6. The engineered immune cell according to claim 5, **characterized in that** the engineered immune cell is obtained by introducing the T cell receptor into an immune cell by means of transduction with a viral vector or a non-viral vector, a CRISPR/Cas9 system, a transposon system, or transfection with mRNA; the engineered immune cell comprises a T cell or a NK cell.

7. A TCR bispecific protein, **characterized in that**, the TCR bispecific protein consists of an antibody targeting a surface receptor molecule on the cell membrane of an immune cell, and an extracellular membrane region of the T cell receptor according to any of claims 1-3, and binds to both an immune cell and a tumor cell;
the immune cell comprises a T cell or a NK cell;
the antibody against a surface receptor molecule on a cell membrane of an immune cell comprises an scFv or Fab antibody fragment.

8. The TCR bispecific protein according to claim 7, **characterized in that**, the surface receptor molecule on a cell membrane of an immune cell is CD3, CD4, CD8, CD16a, and/or NKG2D.

9. The TCR bispecific protein according to claim 7, **characterized in that**, the extracellular membrane region of the T cell receptor recognizes and binds to an antigen on a tumor cell.

10. An mRNA preparation of a TCR bispecific protein, **characterized in**, the mRNA preparation comprises the TCR bispecific protein of any of claims 7-9 and expresses TCR bispecific protein *in vivo* using an mRNA technology.

11. Use of the T cell receptor of any of claims 1-3, the nucleic acid molecule of claim 4, the engineered immune cell of any of claims 5-6, the TCR bispecific protein of any of claims 7-9, or the mRNA preparation of a TCR bispecific protein of claim 10, in the manufacture of a medicament for preventing and/or treating a disease or disorder expression AFP.

12. The use according to claim 11, **characterized in that**, the medicament further comprises a TCR protein drug;
the TCR protein drug is prepared from a multispecific T cell complex prepared from the T cell receptor of any of claims 1-3;
wherein the multispecific T cell complex comprises a TCR bispecific antibody, a TCR trispecific antibody, a TCR tetraspecific antibody;
and comprises a multispecific T cell complex formed from a T cell receptor, a TCR bispecific antibody, a TCR trispecific antibody, or a TCR tetraspecific antibody linked to a nuclear drug, a toxin, a fluorescein, and an antibody fragment.
